(19) **Europäisches Patentamt**

**European Patent Office**

**Office européen des brevets**

(11) **EP 1 358 894 A1**

(12) **EUROPEAN PATENT APPLICATION**

(43) Date of publication:
**05.11.2003 Bulletin 2003/45**

(51) Int Cl.⁷: **A61L 15/42**, A61L 15/46

(21) Application number: **02009937.0**

(22) Date of filing: **03.05.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **SCA Hygiene Products AB**
**405 03 Göteborg (SE)**

(72) Inventors:
• **Lagerstedt-Eidrup, Marie-Louise**
**427 42 Billdal (SE)**

• **Lindström, Äsa**
**417 16 Göteborg (SE)**
• **Forsgren-Brusk, Ulla**
**435 43 Pixbo (SE)**

(74) Representative: **Hammond, Andrew David et al**
**Ström & Gulliksson IP AB**
**Sjöporten 4**
**417 64 Göteborg (SE)**

(54) **Absorbent article with an absorbent body with improved odor performance**

(57)     An absorbent article such as a diaper, sanitary napkin, incontinence protector or the like, comprising an absorbent body enclosed between a liquidimpermeable backing sheet and a liquidpermeable topsheet, characterised in that the absorbent body includes a water-absorbent, predominantly open-celled crosslinked acid-functional addition polymer foams comprising at least one odor control means that controls odor formation on contact with body fluids and is selected from the group consisting of

(a) compounds containing anhydride groups,
(b) compounds containing acid groups,
(c) cyclodextrins,
(d) biocides
(e) surfactants having a HLB value of less than 11

(f) odor adsorbing agents such as zeolites, clay, activated carbon, silica, activated alumina
(g) microorganisms which exhibit antagonistic properties against other unwanted microorganisms
(h) pH-buffering systems
(i) chelating agents

**Description**

TECHNICAL FIELD

This invention relates to an absorbent article such as a diaper, sanitary napkin or an incontinence protector, comprising an absorbent body enclosed between a liquid impermeable backing sheet and a liquid permeable topsheet.

BACKGROUND

Water-absorbent, predominantly open-celled foams based on crosslinked polyacrylates are known, cf. EP-B-0 858 478, WO-A-99/44648 and WO-A-00/52087. They are prepared for example by foaming a polymerizable aqueous mixture containing at least 50 mol% of neutralized acidfunctional monoethylenically unsaturated monomers, crosslinkers and at least one surfactant and subsequently polymerizing the foamed mixture. Foaming of the polymerizable mixture can be effected for example by dispersing fine bubbles of a gas which is inert toward free radicals or by dissolving such a gas under elevated pressure in the polymerizable mixture and decompressing the mixture. The water content of the foams is adjusted to 1-60% by weight for example. The foam are used for example in hygienic articles to acquire and store body fluids. Unpleasant odors may develop in the presence of body fluids when the hygiene articles are used. The development of unpleasant odors from body fluids as sweat, menstrual blood, vaginal discharges, or urine are basically due to two different causes: a) malodorous chemical compounds already contained in the body fluids; and b) malodorous chemical compounds produced by the bacterial metabolism when the bacteria come in contact with body fluids for a prolonged period of time.

WO-A-00/66187 discloses superabsorbent polymer particles comprising a homogeneous distribution throughout the particles of compounds that effect odor control on contact with body fluids. Suitable compounds of this type are cyclodextrins, amphoteric surfactants, water-insoluble phosphates and triclosan.

WO-A-00/76559 discloses a mixture of a water-absorbent polymer and an organic iodine compound. The mixture contains 0.001 to 5% by weight of an iodine compound as a microbicide. The mixtures are present in the form of fine solid particles.

WO-A-01/41819 discloses a particulate mixture containing water-absorbent crosslinked polymers and also from 0.1 to 1000 ppm of a sparingly water-soluble or water-insoluble silver salt or colloidal silver. When hygiene articles containing such mixtures as absorbents for body fluids are used, silver and silver salts effect an odor control.

WO-A-01/68156 discloses hydrogel-forming polymers providing enhanced liquid permeability over the prior art hydrogels described therein and also improved odor control properties. Such hydrogels contain 0.05 to 100% by weight of aluminosilicate, based on polymer.

DESCRIPTION OF THE INVENTION

It is an object of the present invention to provide absorbent articles such as diapers, sanitary napkins, incontinence protectors or the like comprising an absorbent body enclosed between a liquid impermeable backing sheet and a liquid permeable topsheet wherein the absorbent body includes a crosslinked acid-functional addition polymer foam which is treated with an odor-controlling agent. Such absorbent articles provide improved odor and absorption performance.

We have found that this object is achieved according to the invention by an absorbent article such as a diaper, sanitary napkin, incontinence protector or the like, comprising an absorbent body enclosed between a liquid impermeable backing sheet and a liquid permeable topsheet, wherein the absorbent body includes a body fluid - absorbent, predominantly open-celled crosslinked acid-functional addition polymer foam comprising at least one odor control means that controls odor formation on contact with body fluids and is selected from the group consisting of

    (a) compounds containing anhydride groups,
    (b) compounds containing acid groups,
    (c) cyclodextrins,
    (d) biocides
    (e) surfactants having a HLB value of less than 11
    (f) odor adsorbing agents such as zeolites, clay, activated carbon, silica, activated alumina
    (g) microorganisms which exhibit antagonistic properties against other unwanted microorganisms
    (h) pH-buffering systems
    (i) chelating agents

Water-absorbent, predominantly open-celled crosslinked acid-functional polymer foams are known from the prior art cited at the beginning, cf. EP-B-0 858 478 page 2 line 55 to page 10 line 54, WO-A-99/44648 page 4 line 41 to page 27 line 42 and WO-A-00/52087 page 5 line 32 to page 28 line 45. They are also known as hydrogel foams and are obtainable for example by first preparing a polymerizable aqueous mixture containing

- from 10 to 80 % by weight of acid -functional monoethylenically unsaturated monomers which are partially neutralized, for example at least 20mol% neutralized,
- optionally up to 50% by weight of other monoethylenically unsaturated monomers,
- from 0.001 to 5% by weight of crosslinker,
- at least one initiator,
- from 0.1 to 20% by weight of at least one surfactant,
- optionally a solubilizer and
- optionally thickeners, foam stabilizers, polymerization regulators, fillers and/or nucleators.

The polymerizable aqueous mixture is foamed either by dispersing fine bubbles of a gas which is inert toward free radicals or by dissolving an inert gas under a pressure of from 2 to 400 bar and then decompressing the mixture to atmospheric. The foamed mixture is then in either case polymerized to form a hydrogel foam. This method makes it possible to obtain foam articles in any shape, although preference is given to blocks from which foam webs or sheets for example from 0.5 to 10 mm in thickness can be cut, and also to sheets, webs or films. The thus obtainable water-absorbable, predominantly open-celled crosslinked addition polymer foams can be used directly in absorbent articles or postcrosslinked. To postcrosslink the foam articles, they are initially treated with a solution of a crosslinker, for example of a polyhydric alcohol such as propylene glycol or butylene glycol, bisepoxides, or polyglycidyl compounds and the foams are heated to temperatures of for example 120-200°C to postcrosslink the surface.

Useful acid-functional monoethylenically unsaturated monomers include for example acrylic acid, methacrylic acid, acrylamidopropanesulfonic acid or mixtures thereof. Particular preference is given to the use of acrylic acid as a monomer to prepare water-absorbent addition polymers. The acid-functional compounds are usually neutralized with the aid of aqueous sodium hydroxide solution or potassium hydroxide solution. Water-absorbent polymers can also be prepared by polymerizing the acid-functional monomers in the presence of natural products such as starch, cellulose, cellulose derivatives or degradation products of starch such as oxidized starch, enzymatically degraded starch or in the presence of acids or bases of destructured starch. Graft polymers are formed. The polymerization is always effected in the presence of at least one crosslinker, one initiator, and one surfactant. These materials are present in the polymerizable aqueous mixture which is foamed for example by the beaten foam method (dispersing of fine bubbles of an inert gas into the polymerizable mixture) or by dissolving for example carbon dioxide in the polymerizable aqueous mixture under a pressure of for example 12 bar and decompressing this mixture to atmospheric. The flowable foam thus prepared can then be transferred for example onto a belt having side walls or into molds and polymerized into webs, sheets or blocks and subsequently dried. The water content of the foams has a major influence on their flexibility. It is generally in the range from 1 to 60% by weight. Foams having particularly high flexibility are obtained when at least 20 mol% of the acid groups of water-absorbent crosslinked acid-functional polymer foams have been neutralized with alkanolamines, cf. WO-A-00/52087, page 25 line 1 to page 26 line 10. The degree of neutralization of the carboxyl groups of the hydrogel foams is for example in the range from 40 to 95 mol% and preferably in the range from 55 to 85 mol%. By predominantly open celled is meant that at least 80% of the hydrogel foam is open celled. The hydrogel foams are preferably 100% open celled.

The water-absorbent, predominantly open-celled crosslinked acid-functional polymer foam has for example a density of from 0.001 to 0.9 and preferably of from 0.05 to 0.5 g/cm3, a water absorption capacity of at least 5 g/g and a free absorption rate (FAR) of from 4.0 to 100 g/g sec for a 0.9% by weight aqueous sodium chloride solution and a vertical wicking time (VWT= time for 0.9% by weight aqueous sodium chloride solution to advance vertically in a foam) of from 0.2 to 120 seconds for a height of 4 cm.

The hydrogel foam is used as an absorbent body, for example an absorbent layer in an absorbent article. An absorbent article such as a diaper, a sanitary napkin or an incontinence protector comprises an absorbent body enclosed between a liquid impermeable backing sheet and a liquid permeable topsheet. The absorbent body preferably comprises one acquisition layer lying proximal to the liquid pervious topsheet and at least one distribution and/or storage layer lying proximal to the backsheet.

The hydrogel foam can act in the absorbent article for example to acquire the liquid. In such case, the hydrogel foam layer is an acquisition layer. The distribution and/or storage layer is for example made of cellulosic fluff pulp mixed with superabsorbent particles. The superabsorbent particles are polymers, such as for example polymers of polyacrylic acid. Conventional superabsorbent particles have a degree of neutralisation of about 70%. Another example of suitable superabsorbent particles are polymers of polyacrylic acid having a degree of neutralisation of between 20 and 50%. Superabsorbent particles having a degree of neutralisation of between 20% and 50% hamper bacterial growth and bad odours due to a lower pH than conventional superabsorbent particles.

The hydrogel foam can also act in the absorbent article to distribute and/or store body fluids. In such case, the hydrogel foam layer is a distribution and/or storage layer. In such case, the acquisition layer is for example made of an open bulky fiber structure with large capillaries, for example of synthetic fibers.

Another possibility is that the whole absorbent body is made of the hydrogel foam. The liquid acquisition portion and the liquid storage portion are an integrated unit wherein the liquid storage portion comprises a foam which may be same or different from the foam in the liquid acquisition portion. It is for example possible to have different portions having different degree of crosslinking, preferably having higher crosslinking degree closest to the topsheet in the absorbent article and a lower degree of crosslinking closest to the backsheet in the absorbent article. If the whole absorbent body is made of a hydrogel foam, it is also possible that different layers of the hydrogel foams are produced separately and then the different layers are applied on top of each other.

To inhibit any unpleasant odor emanating from body fluids absorbed in absorbent articles by hydrogel foams, the invention provides an absorbent article such as a diaper, sanitary napkin, incontinence protector or the like, comprising an absorbent body enclosed between a liquid impermeable backing sheet and a liquid permeable topsheet, wherein the absorbent body includes an acquisition layer lying proximal to the liquid pervious topsheet and at least one distribution and/or storage layer lying proximal to the backsheet. The absorbent body includes a water-absorbent, predominantly open-celled crosslinked acid-functional addition polymer foam comprising at least one odor control means that controls odor formation on contact with body fluids and is selected from the group consisting of

    (a) compounds containing anhydride groups,
    (b) compounds containing acid groups,
    (c) cyclodextrins,
    (d) biocides
    (e) surfactants having a HLB value of less than 11
    (f) odor adsorbing agents such as zeolites, clay, activated carbon, silica, activated alumina
    (g) microorganisms which exhibit antagonistic properties against other unwanted microorganisms
    (h) pH-buffering systems
    (i) chelating agents.

The backsheet is liquid impermeable and is preferably made of a film layer, a film laminate or a film- and nonwoven laminate. A suitable backsheet is for example a breathable backsheet. In addition to acting as a liquid barrier, a breathable backsheet permits the transfer of moisture vapour, preferably both vapour and air through it and thus allows the circulation of gases into and out of the backsheet. Suitable moisture vapour permeable layers include two dimensional, planar micro and macro-porous films; macroscopically expanded films; formed apertured films; monolithic films and nonwoven layers.

Suitable two-dimensional porous planar layers are for example Goretex (TM ) or Sympatex (TM ) -materials well known in the art for their application in so-called breathable clothing. Other suitable materials include XMP-1001 of Minnesota Mining and Manufacturing Company and Exxaire XBF-101W, supplied by the Exxon Chemical Company. As used herein the two-dimensional planar layer refers to layers having a depth of less than 1 mm, preferably less than 0.5 mm, wherein the apertures have an average uniform diameter along their length and which do not protrude out of the plane of the layer. The apertured backsheet may be produced using any of the methods known in the art such as described in EP 0 293 482 and the references herein. Suitable apertured formed films include films which have discrete apertures which extend beyond the horizontal plane of the garment facing surface of the layer towards the core thereby forming protuberances. Suitable macroscopically expanded films include films as described in for example US 4 637 819 and US 4 591 523. Suitable monolithic films include Hytrel™, available from DuPont Corporation and other such materials described in Index 93 Congress, Session 7A "Adding value to Nonwovens", J-C. Cardinal and Y. Trouilhet, DuPont de Nemours international S.A, such as Pebax™, available from Elf Atochem (France) and Estane™ available from BF Goodrich (Belgium).

Useful compounds (a) containing anhydride groups include for example maleic anhydride, itaconic anhydride, polymaleic anhydride, polyitaconic anhydride and/or copolymers of maleic anhydride and a C2-to C8-olefin or styrene. These compounds can be added to the aqueous polymerizable mixture before the foam is prepared. The anhydride groups of the odor-controlling agents hydrolyze during foam production, yet still provide effective odor control, since they lower the pH of the hydrogel foam. The pH of the hydrogel foams treated with these agents is for example in the range from 4.0 to 6.5 and preferably is from 4.5 to 5.5.

However, the hydrogel foams are preferably treated with solutions of the compounds of component (a) in an inert solvent which does not react with the anhydride groups. Useful solvents include for example ketones such as acetone or methyl ethyl ketone, dioxane, dimethyl sulfoxide, ethyl acetate, toluene and chloroform.

The solutions of the compounds containing anhydride groups can either be sprayed onto the hydrogel foams or the hydrogel foams can be dipped into a solution of a compound containing anhydride groups. Excess solvent is removed after the foam has been impregnated, advantageously by drying the treated foam, for example at from 20 to 120°C under atmospheric pressure, or the component (a) which are applied to the hydrogel foams are for example from 1 to 10% and preferably from 5 to 8% by weight, based on hydrogel.

Useful polymeric compounds of component (a) are polymaleic anhydride, polyitaconic anhydride and copolymers of maleic anhydride and a C2- to C8-olefin or styrene. The polymers can be prepared for example by free radical polymerization of their underlying monomers in a precipitation polymerization in an inert solvent of the monomers, for example toluene or xylene. The molar masses Mw of the polymers are for example in the range from 500 to 1 million and preferably in the range from 1000 to 250 000.

Useful copolymers include in particular copolymers of maleic anhydride and isobutene, copolymers of maleic anhydride and diisobutene and also copolymers of maleic anhydride and styrene. The comonomers are preferably present in the copolymers in an equimolar ratio, but the copolymers may by way of modification optionally contain further comonomers such as acrylic acid, methacrylic acid, vinyl acetate or acrylic esters of monohydric alcohols having from 1 to 18 carbon atoms.

Useful compounds containing acid groups include for example ascorbic acid, benzoic acid, citric acid, salicylic acid, sorbic acid, adipic acid, malic acid, tartaric acid, lactic acid and water-soluble polymers of monomers containing acid groups. Polymeric compounds include for example homopolymers of monoethylenically unsaturated C3- to C5-carboxylic acids, copolymers of monoethylenically unsaturated C3- to C5-carboxylic acids and/or amphiphilic copolymers of (i) at least one hydrophobic monoethylenically unsaturated carboxylic acids, monoethylenically unsaturated sulfonic acids, monoethylenically unsaturated phosphonic acids or mixtures thereof.

Individual polymeric compounds of group (b) are for example polyacrylic acid or polymethacrylic acid, copolymers of acrylic acid and methacrylic acid, copolymers of acrylic acid and maleic acid, copolymers of methacrylic acid and maleic acid and also the abovementioned amphiphilic copolymers, which are obtainable for example by free radical polymerization in an aqueous medium of at least one hydrophobic monomer (i) and of a hydrophilic monomer (ii).

Examples of hydrophobic monomers of group (i) are: styrene, methylstyrene, ethylstyrene, acrylonitrile, methacrylonitrile, C2- to C18-olefins, esters of monoethylenically unsaturated C3- to C5-carboxylic acids and monohydric alcohols, vinyl alkyl ethers, vinyl esters or mixtures of two or more of the compounds mentioned. Preferred compounds of this group of monomers are styrene, isobutene, diisobutene, vinyl acetate, methyl acrylate, ethyl acrylate, propyl acrylate, isopropyl acrylate, n-butyl acrylate, isobutyl acrylate, tert-butyl acrylate and 2-ethylhexyl acrylate.

Examples of hydrophilic monomers of group (ii) are: acrylic acid, methacrylic acid, maleic acid, itaconic acid, vinylsulfonic acid, 2-acrylamidomethylpropanesulfonic acid, acrylamidopropane-3-sulfonic acid, 3-sulfopropylacrylic acid, 3-sulfopropylmethacrylic acid, styrenesulfonic acid, vinylphosphonic acid and mixtures thereof. When the amphiphilic copolymers are not sufficiently water soluble, they are converted into the more readily soluble salt form by a partial neutralization with bases, such as sodium hydroxide solution, potassium hydroxide solution, ammonia, amines or alkanolamines. However, care must be taken here to ensure that the pH value of the partially neutralized aqueous solutions does not exceed a value of 6.5. The pH of these solutions is usually below 5.0. The average molecular weight Mw of the homo- and copolymers is for example in the range from 1000 to 1 million, preferably in the range from 1500 to 150 000.

Particularly preferred amphiphilic copolymers contain

(i) from 95 to 40% by weight of isobutene, diisobutene, styrene or mixtures thereof, and
(ii) from 5 to 60% by weight of acrylic acid, methacrylic acid, maleic acid, monoesters of maleic acid with monohydric alcohols containing from 1 to 18 carbon atoms.

These copolymers may optionally contain further comonomers in polymerized form. Other copolymers useful as odor control means are preparable by graft polymerization of monoethylenically unsaturated acids onto natural or synthetic polymers. Useful grafting bases for such polymers include for example starch, cellulose, derivatives of cellulose such as carboxymethylcellulose, oxidatively, hydrolytically or enzymatically degraded starches, galactomannans, alginates, polyamines, polyvinyl alcohol, polyamides and hydrophilic polyesters. This group of compounds also includes hydrolyzed graft polymers of acrylonitrile on starch whose nitrile groups have been hydrolyzed to carboxyl groups. Further useful carboxyl-containing polymers are obtainable by hydrolysis of vinyl acetate-acrylic ester copolymers, polymers containing acrylonitrile groups or polymers containing acrylamide groups.

Hydrogel foams containing a compound of component (b) for odor control are prepared by treating the hydrogel foams with solutions of the compounds containing acid groups. Useful solvents include for example water, alcohols, ketones or ethers and also mixtures thereof. The acid groups of the polymers may optionally be partially neutralized, in which case the pH of the solutions should be not more than 6.5 and preferably below 5.0. The hydrogel foams are treated for example by dipping the foams into a solution containing at least one compound of group (b) or by spraying such a solution onto the foam which is to be provided with the odor-inhibiting compound. The amount of compounds of component (b) which is contacted with the hydrogel foam is for example in the range from 1 to 20% and preferably in the range from 5 to 10% by weight, based on the foam.

Component (c) may be for example any cyclodextrin such as α-cyclodextrin, β-cyclodextrin or γ-cyclodextrin or mixtures or derivatives thereof. The cyclodextrinization of the hydrogel foams is effected similarly to the use of component (b) described above, by dipping the foams into a solution of at least one cyclodextrin or by spraying a solution of a cyclodextrin.

Component (d) may be any biocide. By biocide is meant any substance intended to destroy, deter, render harmless, prevent the action of, or otherwise exert a controlling effect on any harmful organism by chemical or biological means.. Examples of biocides are quaternary ammonium compounds, phenols, amides, nitro compounds, iodine compounds and also silver and zinc compounds. Ammonium compounds whose use is preferred are cetylpyrridinium chloride, lauryltrimethylammonium chloride, stearyltrimethylammonium chloride, benzalkonium chlorides (mixture of alkyldimethylbenzylammonium chlorides, where alkyl = C8H17-to C18H37-), benzethonium chloride, chlorhexidine hydrochloride (=1,6-di(4-chlorophenyldiguanidino)-hexane dihydrochloride), chlorhexidine gluconate and triclosan. Useful phenols include for example isopropylmethylphenol, p-chlorophenol, resorcinol, resorcinol monoacetate, sodium phenolsulfonate, di-tert-butylphenol, hydroquinone and chlorocresol. Preferred amides include for example 2,4-imidazolidinedione (hydantoin), 3,4,4'-trichlorocarbanilide, 3-trifluoromethyl- 4,4'-dichlorocarbanilide and undecylenamide MEA. An example of a nitro compound is 2-bromo-2-nitro-2,3-propanediol (Bronopol).

Useful biocides for odor inhibition also include iodine compounds, for example 3-iodo-2-propynyl-N-butylcarbamate, 4-methylphenyl diiodomethyl sulfone, polyvinylpyrrolidone-iodine complexes and starch-iodine complexes and also colloidal silver, silver and zinc compounds, for example silver oxide, silver zeolite silver benzoate and zinc chloride. Chitin and parabens are also examples of biocides.

Any water-insoluble biocide may be dispersed in water and the dispersion thus obtainable used for treating the hydrogel foams. Bactericides are used for example in amounts from 1 to 10 000 and preferably from 100 to 600 ppm, based on hydrogel foam.

Component (e) may be any surfactant having an HLB value below 12 and preferably of not more than 10. HLB refers to the hydrophilic/lipophilic balance of compound, cf. N.C. Griffin, Journal of Society of Cosmetic Chemist, volume 1, 311 (1950). Typical products of this kind are ethoxylated sorbitan monooleate having a HLB value of 10 and sorbitan monooleate having an HLB value of 4.3. Useful compounds of this group further include sorbitan monostearate and ethoxylated sorbitan monostearate. They are used for example in amounts ranging from 0.05 to 5% and preferably from 0.1 to 2% by weight, based on the foam.

Component (f) is an odor adsorbing agent such as a zeolite, clay, activated carbon, silica or activated alumina. If component (f) is a zeolite, the zeolite may be a naturally derived zeolite or a synthetically manufactured zeolite. Suitable zeolites for use herein include for example zeolite A, zeolite P, zeolite Y, zeolite X, zeolite DAY, zeolite ZSM-5, or mixtures thereof. Preferred cations may be selected from group I and II metals and may also include heavy metals such as for example zinc, silver and copper. The zeolite is preferably hydrophobic. This is typically achieved by increasing the molar ratio of the SiO2 to AlO2 content such that the ratio of x to y is at least 1, preferably from 1 to 500, most preferably from 1 to 6. Example of a suitable clay, is bentonite clay.

Component (g) is a microorganism or a mixture of microorganisms which exhibit antagonistic properties against other unwanted microorganisms. When the absorbent article is worn, antagonistic properties against undesirable strains of microorganisms, for example from the families Enterobacteriaceae, Micrococcaceae, Pseudomonadaceae and Ascomycetes and the genera Streptococcus, Gardnerella and Candida, is achieved. The antagonistic bacteria is preferably lactic acid producing bacteria from the genera Lactobacillus, Lactococcus or Bacillus.

Component (h) is a pH-buffering system. Suitable pH-buffering systems may be made from said acids mentioned under (b) and their corresponding salts.

Component (i) is a chelating agent. Suitable chelating agents can be selected from the group consisting of amino carboxylates, amino phosphonates, polyphosphates, hydroxycarboxylic acids, polyfunctionally-substituted aromatic chelating agents, and mixtures thereof.

The treatment of hydrogel foams with at least one compound of components (a) to (i) leads in contrast to a particulate hydrogel without foam structure to a product which when used as an absorbent body in an absorbent article exhibits a surprisingly improved odor inhibition following the absorption of body fluids.

Unless the context suggests otherwise, the %ages in the examples are by weight.

Determination of monomer foam density:

100 ml of the monomer foam are introduced into a graduated cylinder and the weight of the foam volume is determined. Dividing the weight found in g by 100 provides the density of the foam in g/cm3.

Determination of the polymer foam density:

The density of hydrogel foams is determined gravimetrically. A uniform foam layer having a defined thickness in the range from 3 to 5 mm is cut for example with a sharp knife to obtain square shapes having an edge length of 5 cm. The samples are weighed and the weight obtained is divided by the volume calculated from the dimensions.

Determination of absorption capacity:

The absorption capacity of the superabsorbent foam in terms of water per gram of superabsorbent is determined on pieces of foam having a thickness of 3 mm and each weighing 1 g. The absorption capacity is here tested by the teabag test. The liquid used is 0.9% by weight sodium chloride solution. 1 g of the foam material is introduced into a teabag, which is then sealed. Care must be taken to ensure that the teabag offers sufficient room for complete swelling. The teabag is then immersed for a certain period, for example 30 min, into the liquuid and weighed back after a drip-off time of for example 10 minutes. The blank is determined by immersing the teabag without superabsorbent in the solution and determining the weight of the teabag under the conditions described above. The absorption capacity then follows from the following equation (1):

$$\text{Absorption capacity} = (G_{TS} - G_T) / G_S$$

Where

$G_{TS}$ is the weight of the teabag with superabsorbent foam
$G_T$ is the weight of the teabag in the blank test and
$G_S$ is the starting weight of the superabsorbent foam

Determination of absorption rate:

The free absorption rate (hereinafter referred as FAR) is found by cutting out, using a sharp knife, rectangular samples weighing 1 g from doam layers having a uniform thickness of 3 mm. These samples are placed in a Petri dish and 20 g of 0.9% sodium chloride solution are poured over. A stopwatch is used to determine the time required for the foam sample to completely absorb the 0.9% sodium chloride solution. The absorption rate FAR in g/gsec is calculated from the following equation (2):

$$\text{FAR} = 20 \text{ g} / (1\text{g} \times \text{measured time in seconds}) \tag{2}$$

Droplet absorption rate (DAR):

A droplet of 0.9% sodium chloride is placed on the surface of the foam layer and the time is taken for the droplet to completely disappear into the foam. The same process is repeated for the second side of the foam layer and the two values are identified as above and below.

Vertical wicking time (VWT):

A petri dish (10 cm in diameter and 1 cm in height) is filled with 0.9% sodium chloride solution up to depth of 0.5 cm. A glass tube 1 cm in diameter and 15 cm in length is then sited a short distance above the base of the dish. A foam strip 6 cm in length having a square base area of 5 x 5 mm is marked at 2.4 and 6 cm and placed inside the glass tube in the liquid. The time measurement is started. The time in seconds taken to reach the respective mark is determined.

Example 1

The following components were mixed in a beaker using a magnetic stirrer.

- 348.55 g of acrylic acid (4.84)
- 135.51 g of 37.3% sodium acrylate solution in water (0.54 mol)
- 28.00 g of polyethylene glycol diacrylate of polyethylene glycol of molar mass 400.

- 21.33 g of a 15% aqueous solution of an addition product of 80 mol of ethylene oxide with 1 mol of a linear saturated C16C18 fatty alcohol.
- 65.70 g of water

5% of maleic anhydride and, with ice-cooling, 400.90 g (2.69 mol) of triethanolamine were added to this solution in such a way that the internal temperature did not rise above 16oC. The resulting solution was transferred into a pressure vessel and saturated therein with carbon dioxide under a pressure of 12 bar for 25 min. Under pressure, 26.67 g of a 3% aqueous solution of 2,2'-azobis(2-amidinopropane) dihydrochloride were added and mixed in using a fast stream of carbon dioxide until the mixture was homogeneous. Carbon dioxide was then passed through the reaction mixture for a further 5 min. The saturated reaction mixture was forced under a pressure of 12 bar through a 1 mm diameter nozzle to form a finely celled free-flowing foam.

The monomer foam obtained was placed on an A3 size glass plate having edges 3 mm in height and covered with a second glass plate. The foam sample was irradiated synchronously from both sides with two UV/VIS lamps (Höhnle UV 1000) for 4 minutes.

The foam layer obtained was completely dried in a vacuum oven at 70oC. To determine the properties, a portion of the foam was then adjusted to a moisture content of 5% by spraying with water.

Solids content of reaction mixture: 81.04%
Degree of neutralization: 60 mol%
Monomer foam density: 0.18 g/cm3
Polymer foam density: 0.19 g/cm3
Foam structure: homogeneous, completely open celled, no skin

When the foam thus prepared is exposed to urine, pH control prevents the release of malodorous compounds.

Example 2

The following components were mixed in a beaker using a magnetic stirrer.

- 248.55 g of acrylic acid (4.84 mol)
- 135.51 g of 37.3% sodium acrylate solution in water (0.54 mol)
- 28.00 g of polyethylene glycol diacrylate of polyethylene glycol of molar mass 400
- 21.33 g of a 15% aqueous solution of an addition product of 80 mol of ethylene oxide with 1 mol of a linear saturated C16C18 fatty alcohol
- 65.70 g of water

With ice-cooling, 400.90 g (2.69 mol) of triethanolamine were added to this solution in such a way that the internal temperature did not rise above 16oC. The resulting solution was transferred into a pressure vessel and saturated therein with carbon dioxide under a pressure of 12 bar for 25 min. Under pressure, 26.67 g of a 3% aqueous solution of 2,2'-azobis (2-amidinopropane) dihydrochloride were added and mixed in using a fast stream of carbon dioxide until the mixture was homogeneous. Carbon dioxide was then passed through the reaction mixture for a further 5 min. The saturated reaction mixture was forced under a pressure of 12 bar through a 1 mm diameter nozzle to form a finely celled free-flowing foam.

The monomer foam obtained was placed on an A3 size glass plate having edges 3 mm in height and covered with a second glass plate. The foam sample was irradiated synchronously from both sides with two UV/VIS lamps (Höhnle UV 1000) for 4 minutes.

The foam layer obtained was completely dried in a vacuum oven at 70oC. To determine the properties, a portion of the foam was then adjusted to a moisture content of 5% by spraying with water. The surface of the hydrogel foam had 5% of maleic anhydride applied to it by spraying with a 20% solution of maleic anhydride in acetone and drying the foam overnight.

Solids content of reaction mixture: 81.04%
Degree of neutralization: 60 mol%
Monomer foam density: 0.18 g/cm3
Polymer foam density: 0.19 g/cm3
Foam structure: homogeneous, completely open celled, no skin

When the foam thus prepared is exposed to urine, pH control prevents the release of malodorous compounds for a prolonged period.

Example 3

Example 1 was repeated with the single exception that the degree of neutralization of the hydrogel foam was adjusted to 45 mol%. The lower degree of neutralization than in example 1 and the impregnation of the hydrogel foam with maleic anhydride led to effective odor control on prolonged contact with urine.

Example 4

Example 2 was repeated with the single exception that the degree of neutralization of the hydrogel foam was adjusted to 45 mol%. The lower degree of neutralization than in example 2 and the impregnation of the hydrogel foam with maleic anhydride led to effective odor control on prolonged contact with urine.

Example 5

Example 1 was repeated with the exceptions that maleic anhydride was replaced by the same amount of polymaleic anhydride having a molar mass Mw of 12000 and the degree of neutralization of the hydrogel foam was adjusted to 45 mol%. The hydrogel foams thus prepared provided in hygiene articles an effective control of the odor which otherwise emananctes from the bacteria following the absorption of body fluids.

Example 6

Example 2 was repeated with the exceptions that maleic anhydride was replaced by the same amount of polymaleic anhydride having a molar mass Mw of 12000 and the degree of neutralization of the hydrogel foam was adjusted to 45 mol%. The hydrogel foams thus prepared provided in absorbent articles an effective control of the odor which otherwise emananctes from the bacteria following the absorption of body fluids.

Example 7

The following compponents were mixed in a beaker using a magnetic stirrer.

- 348.55 g of acrylic acid (4.84)
- 135.51 g of 37.3% sodium acrylate solution in water (0.54 mol)
- 28.00 g of polyethylene glycol diacrylate of polyethylene glycol of molar mass 400
- 21.33 g of a 15% aqueous solution of an addition product of 80 mol of ethylene oxide with 1 mol of a linear saturated C16C18 fatty alcohol
- 65.70 g of water

With ice-cooling, 400.90 g (2.69 mol) of triethanolamine were added to this solution in such a way that the internal temperature did not rise above 16oC. The resulting solution was transferred into a pressure vessel and saturated therein with carbon dioxide under a pressure of 12 bar for 25 min. Under pressure, 26.67 g of a 3% aqueous solution of 2,2'-azobis (2-amidinopropane) dihydrochloride were added and mixed in using a fast stream of carbon dioxide until the mixture was homogeneous. Carbon dioxide was then passed through the reaction mixture for a further 5 min. The saturated reaction mixture was forced under a pressure of 12 bar through a 1 mm diameter nozzle to form a finely celled free-flowing foam.

The monomer foam obtained was placed on an A3 size glass plate having edges 3 mm in height and covered with a second glass plate. The foam sample was irradiated synchronously from both sides with two UV/VIS lamps (Höhnle UV 1000) for 4 minutes.

The foam layer obtained was completely dried in a vacuum oven at 70oC. To determine the properties, a portion of the foam was then adjusted to a moisture content of 5 % by spraying with water.

Solids content of reaction mixture: 81.04%
Degree of neutralization: 60 mol%
Monomer foam density: 0.18 g/cm3
Polymer foam density: 0.19 g/cm3
Foam structure: homogenous, completely open celled, no skin

The β-cyclodextrin used for odor control is present in the polymerized foam in a uniform distribution. An absorbent article comprising an absorbent body of this foam provides odor inhibition following the absorption of body fluids.

Example 8

Example 7 is repeated except that 1000 ppm of silver zeolite are used instead of cyclodextrin. An absorbent article comprising an absorbent body of this hydrogel foam provides odor inhibition following the absorption of urine.

Example 9

The following components were mixed in a beaker using a magnetic stirrer.

- 348.55 g of acrylic acid (4.84)
- 135.51 g of 37.3% sodium acrylate solution in water (0.54 mol)
- 28.00 g of polyethylene glycol diacrylate of polyethylene glycol of molar mass 400
- 21.33 g of a 15% aqueous solution of an addition product of 80 mol of ethylene oxide with 1 mol of a linear saturated C16C18 fatty alcohol
- 65.70 g of water

With ice-cooling, 400.90 g (2.69 mol) of triethanolamine were added to this solution in such a way that the internal temperature did not rise above 16oC. The resulting solution was transferred into a pressure vessel and saturated therein with carbon dioxide under a pressure of 12 bar for 25 min. Under pressure, 26.67 g of a 3% aqueous solution of 2,2'-azobis(2-amidinopropane) dihydrochloride were added and mixed in using a fast stream of carbon dioxide until the mixture was homogeneous. Carbon dioxide was then passed through the reaction mixture for a further 5 min. The saturated reaction mixture was forced under a pressure of 12 bar through a 1 mm diameter nozzle to form a finely celled free-flowing foam.

The monomer foam obtained was placed on an A3 size glass plate having edges 3 mm in height and covered with a second glass plate. The foam sample was irradiated synchronously from both sides with two UV/VIS lamps (Höhnle UV 1000) for 4 minutes.

The foam layer obtained was completely dried in a vacuum oven at 70oC. To determine the properties, a portion of the foam was then adjusted to a moisture content of 5 % by spraying with water.

Solids content of reaction mixture: 81.04%
Degree of neutralization: 60 mol%
Monomer foam density: 0.18 g/cm3
Polymer foam density: 0.19 g/cm3
Foam structure: homogenous, completely open celled, no skin

A 10% solution of β-cyclodextrin in methanol was prepared and sprayed onto the surface of the foam thus prepared and the hydrogel foam, which contained β-cyclodextrin at its surface, was dried overnight at room temperature. An absorbent article comprising an absorbent body of this hydrogel foam provides odor inhibition following the absorption of urine.

Example 10

Example 9 was repeated with the single exception that the surface of the foam was sprayed with an aqueous dispersion containing 1000 ppm of silver oxide instead of with β-cyclodextrin. The use of a solvent mixture makes it possible to adjust the degree of penetration of the coating mixture. The dispersion-sprayed foam is dried at room temperature overnight. The silver oxide adheres to the surface of the foam. An absorbent article comprising an absorbent body of this hydrogel foam provides odor inhibition following the absorption of urine.

Example 11

Example 9 was repeated with the single exception that the surface of the foam was sprayed with an aqueous dispersion containing 1000 ppm of silver benzoate instead of with β-cyclodextrin. The hydrogel foam thus prepared was dried at room temperatur overnight. The silver benzoate was situated in the interior and on the surface of the hydrogel foam. An absorbent article comprising an absorbent body of this hydrogel foam provides odor inhibition following the absorption of urine.

Example 12

Example 9 was repeated except that an aqueous dispersion containing 200 ppm of zinc chloride was sprayed onto the surface of the hydrogel foam. The foam, which contained the zinc chloride in its interior and on the surface, was dried overnight. An absorbent article comprising an absorbent body of this hydrogel foam provides odor inhibition following the absorption of urine.

Example 13

Example 9 was repeated with the single exception that instead of β-cyclodextrin an aqueous dispersion of 1% of sorbitan monooleate was sprayed onto the foam.

Example 14

The foam prepared according to example 9 was not treated with β-cyclodextrin but instead sprayed with a 1% dispersion of cetylpyrridinium chloride in methanol and thereafter dried at room temperature. The cetylpyrridinium chloride biocide is on the surface of the hydrogel foam.

Example 15

The foam prepared according to example 9 was not treated with β-cyclodextrin But instead treated with a 5% solution of citric acid in methanol and thereafter dried at room temperature overnight. An absorbent article comprising an absorbent body of this hydrogel foam provides odor inhibition following the absorption of urine.

Example 16

The foam prepared according to example 9 was not treated with β-cyclodextrin but instead treated with a 5% solution of triclosan in methanol and thereafter dried at room temperature overnight. The triclosan remained on the surface of the hydrogel foam. An absorbent article comprising an absorbent body of this hydrogel foam provides odor inhibition following the absorption of urine.

Example 17

Example 9 was repeated with the exception that the surface of the foam was sprayed with a suspension containing lactic acid producing bacteria. The sprayed foam was dried at room temperature overnight. An absorbent article comprising an absorbent body of this hydrogel foam was produced. Each article contained about $1 \times 10^9$ cfu of such microorganisms. The absorbent article delivered outstanding odor control benefits when in use, typically when coming in contact with bodily fluids.

Example 18

Example 9 was repeated with the exception that the surface of the foam was sprayed with a suspension containing spore forming lactic acid producing bacteria. The sprayed foam was dried at room temperatura overnight. An absorbent article comprising an absorbent body of this hydrogel foam was produced. Each article contained about $1 \times 10^9$ cfu of such microorganisms. The absorbent article delivered outstanding odor control benefits when in use, typically when coming in contact with bodily fluids.

Example 19

Example 9 was repeated with the exception that the surface of the foam was sprayed with a dispersion containing zeolite. The sprayed foam was dried at room temperature overnight. An absorbent article comprising an absorbent body of this hydrogel foam was produced. The absorbent article delivered outstanding odor control benefits when in use, typically when coming in contact with bodily fluids.

**Claims**

1. An absorbent article such as a diaper, sanitary napkin, incontinence protector or the like, comprising an absorbent body enclosed between a liquid impermeable backing sheet and a liquid permeable topsheet,

**characterised in that** the absorbent body includes a water-absorbent, predominantly open-celled crosslinked acid-functional addition polymer foams comprising at least one odor control means that controls odor formation on contact with body fluids and is selected from the group consisting of

(a) compounds containing anhydride groups,
(b) compounds containing acid groups,
(c) cyclodextrins,
(d) biocides
(e) surfactants having a HLB value of less than 11
(f) odor adsorbing agents such as zeolites, clay, activated carbon, silica, activated alumina
(g) microorganisms which exhibit antagonistic properties against other unwanted microorganisms
(h) pH-buffering systems
(i) chelating agents

2. An absorbent article as claimed in claim 1
**characterised in that** the compounds (a) containing anhydride groups are maleic anhydride, itaconic anhydride, polymaleic anhydride, polyitaconic anhydride and/or copolymers of maleic acid and a C2- to C8-olefin or styrene.

3. An absorbent article as claimed in claim 1,
**characterised in that** compounds (b) containing acid groups are ascorbic acid , benzoic acid, citric acid, salicylic acid, sorbic acid, adipic acid, malic acid, tartaric acid, lactic acid and water-soluble polymers of monomers containing acid groups.

4. An absorbent article as claimed in claim 1, **characterised in that** compounds (b) containing acid groups are homopolymers of monoethylenically unsaturated C3- to C5-carboxylic acids, copolymers of monoethylenically unsaturated C3- to C5-carboxylic acids and/or amphiphilic copolymers of (i) at least one hydrophobic monoethylenically unsaturated monomer and (ii) monoethylenically unsaturated carboxylic acids, monoethylenically unsaturated sulfonic acids, monoethylenically unsaturated phosphonic acids or mixtures thereof.

5. An absorbent article as claimed in claim 1, **characterised in that** compounds (a) are maleic anhydride, polymaleic anhydride and/or copymers of maleic anhydride and isobutene, copolymers of maleic anhydride and diisobutene and/or copolymers af maleic anhydride and styrene.

6. An absorbent article as claimed in claim 1, **characterised in that** compounds (b) are homopolymers of acrylic acid, copolymers of acrylic acid and maleic acid and/or copolymers of acrylic acid and methacrylic acid.

7. An absorbent article as claimed in claim 1, **characterised in that** compounds (c) are β-cyclodextrin.

8. An absorbent article as claimed in claim 1, **characterised in that** compounds (d) are silver salts, zinc salts, cetylpyrridinium chloride and/or triclosan.

9. An absorbent article as claimed in claim 1, **characterised in that** compounds (g) are lactic acid producing bacteria from the genera Lactobacillus, Lactococcus or Bacillus.

10. An absorbent article according to any of the claims 1-9,
**characterised in that** the absorbent body includes an acquisition layer lying proximal to the liquid pervious topsheet and at least one distribution and/or storage layer lying proximal to the backsheet.

EP 1 358 894 A1

**European Patent Office**

## EUROPEAN SEARCH REPORT

Application Number

EP 02 00 9937

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 94 22500 A (PROCTER & GAMBLE) 13 October 1994 (1994-10-13) * page 3 * * claims * | 1,2,5,10 | A61L15/42 A61L15/46 |
| X,D | US 6 229 062 B1 (DARLINGTON JR JERALD W ET AL) 8 May 2001 (2001-05-08) * column 2, line 55 - column 3, line 34 * * column 4, line 13-23 * * column 5, line 18-29 * * column 6, line 1-4 * * column 12, line 32-35 * | 1,2,5,10 | |
| X | WO 98 26808 A (TRINH TOAN ;PROCTER & GAMBLE (US)) 25 June 1998 (1998-06-25) * page 25, paragraph 3 - page 26, paragraph 3 * * claims * | 1,2,5,10 | |

TECHNICAL FIELDS SEARCHED (Int.Cl.7)

A61L
A61F

The present search report has been drawn up for all claims

| Place of search | Date of completion of the search | Examiner |
|---|---|---|
| THE HAGUE | 17 October 2002 | Böhm, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

13

European Patent
Office

Application Number

EP 02 00 9937

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 10 (partial) ; 2, 5 (complete)

**LACK OF UNITY OF INVENTION
SHEET B**

Application Number

EP 02 00 9937

The Search Division considers that the present European patent application does not comply with the requirements of unity of invention and relates to several inventions or groups of inventions, namely:

1. Claims: 1,10 (partial.) ; 2,5 (complete.)

   An absorbent article [...] comprising at least one odor control means that controls odor formation on contact with body fluids, these are :

   (a) compounds containing anhydride groups

   - maleic anhydride, itaconic anhydride, polymaleic anhydride, polyitaconic anhydride and/or copolymers (claim 2)

   - maleic anhydride, polymaleic anhydride and/or copolymers, maleic anhydride and isobutene, copolymers of maleic anhydride and diisobutene... (claim 5)

2. Claims: 1,10 (partial.) ; 3,4,6 (complete.)

   An absorbent article [...] comprising at least one odor control means that controls odor formation on contact with body fluids, these are :

   (b) compounds containing acid groups

   - ascorbic acid, benzoic acid, citric acid, salicylic acid, sorbic acid, adipic acid, malic acid, benzoic acid, citric acid, salicylic acid, sorbic acid, adipic acid, tartaric acid, lactic acid and water-soluble polymers of monomers containing acid groups (claim 3)

   - homopolymers of monoethylenically unsaturated C3- to C5-carboxylic acids,... (claim 4)

   - homopolymers of acrylic acid, copolymers of acrylic acid and maleic acid and/or copolymers of acrylic and methacrylic acid (claim 6)

3. Claims: 1,10 (partial.) ; 7 (complete.)

   An absorbent article [...] comprising at least one odor control means that controls odor formation on contact with body fluids, these are :

   (c) a cyclodextrin (beta-cyclodextrin, see claim 7)

4. Claims: 1,10 (partial.) ; 8 (complete.)

**European Patent Office**

**LACK OF UNITY OF INVENTION SHEET B**

**Application Number**

EP 02 00 9937

The Search Division considers that the present European patent application does not comply with the requirements of unity of invention and relates to several inventions or groups of inventions, namely:

An absorbent article [...] comprising at least one odor control means that controls odor formation on contact with body fluids, these are :

(d) a biocide,

- such as silver salts, zinc salts, cetylpyrridinium chloride and/or triclosan (clam 8)

5. Claims: 1,10 (partial.) ; 9 (complete.)

An absorbent article [...] comprising at least one odor control means that controls odor formation on contact with body fluids, these are :

(g) microorganisms which exhibit antagonistic properties against other unwanted microorganisms

- such as lactic acid producing bacteria from the genera Lactobacillus, Lactococcus or Bacillus (claim 9)

6. Claims: 1,10 (partial.)

An absorbent article [...] comprising at least one odor control means that controls odor formation on contact with body fluids, these are :

(e) surfactants having a HLB value of less than 11

7. Claims: 1,10 (partial.)

An absorbent article [...] comprising at least one odor control means that controls odor formation on contact with body fluids, these are :

(f) odor adsorbing agents such as zeolites, clay, activated carbon, silica, activated alumina

8. Claims: 1,10 (partial.)

An absorbent article [...] comprising at least one odor control means that controls odor formation on contact with body fluids, these are :

(h) pH-buffering systems

**European Patent Office**

**LACK OF UNITY OF INVENTION**
**SHEET B**

Application Number

EP 02 00 9937

The Search Division considers that the present European patent application does not comply with the requirements of unity of invention and relates to several inventions or groups of inventions, namely:

9. Claims: 1,10 (partial.)

An absorbent article [...] comprising at least one odor control means that controls odor formation on contact with body fluids, these are :

(i) chelating agents

## ANNEX TO THE EUROPEAN SEARCH REPORT
## ON EUROPEAN PATENT APPLICATION NO.

EP 02 00 9937

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2002

| Patent document cited in search report | | Publication date | Patent family member(s) | | Publication date |
|---|---|---|---|---|---|
| WO 9422500 | A | 13-10-1994 | AU | 693091 B | 25-06-1998 |
| | | | AU | 6447094 A | 24-10-1994 |
| | | | CA | 2157465 A | 13-10-1994 |
| | | | EP | 0691857 A | 17-01-1996 |
| | | | JP | 8508423 T | 10-09-1996 |
| | | | NZ | 263688 A | 27-05-1998 |
| | | | SG | 54200 A | 16-11-1998 |
| | | | US | 5733272 A | 31-03-1998 |
| US 6229062 | B | 08-05-2001 | AU | 4180800 A | 17-11-2000 |
| | | | BR | 0010091 A | 08-01-2002 |
| | | | CN | 1349420 T | 15-05-2002 |
| | | | EP | 1173234 A | 23-01-2002 |
| | | | WO | 0066187 A | 09-11-2000 |
| WO 9826808 | A | 25-06-1998 | AU | 739247 B | 04-10-2001 |
| | | | AU | 5597298 A | 15-07-1998 |
| | | | EP | 0946209 A | 06-10-1999 |
| | | | JP | 2000505692 T | 16-05-2000 |

For more details about this annex : see Official Journal of the European Patent Office, No. 12/82